# EUROPEAN PATENT SPECIFICATION

(11) **EP 1 635 810 B1**
(45) Date of publication and mention of the grant of the patent: **31.03.2010**
(21) Application number: 04743058.2
(22) Date of filing: 23.06.2004
(51) Int. Cl.: A61K 31/202, A61K 31/07, A61K 31/122, A61K 31/015, A61K 31/01

(54) **INFLAMMATORY DISEASE TREATMENT**
BEHANDLUNG VON ENTZÜNDLICHEN ERKRANKUNGEN
TRAITEMENT D'UNE MALADIE INFLAMMATOIRE

(30) Priority: 23.06.2003 GB 0314624
(43) Date of publication of application: 22.03.2006
(73) Proprietor: Advanced Bionutrition (Europe) Limited, Worthing, West Sussex BN11 1QR (GB)
(72) Inventor: CLAYTON, Diane, Advanced Bionutrition (Europe) Ltd, Worthing, West Sussex BN11 1QR (GB); RUTTER, Rebecca, Advanced Bionutrition (Europe) Lt, Worthing, West Sussex BN11 1QR (GB)
(74) Representative: Taylor, Kate Laura
(86) International application number: PCT/GB2004/002707
(87) International publication number: WO 2004/112776

(56) References cited:
- EP-A- 0 699 437
- WO-A-01/24787
- WO-A-98/37874
- WO-A-02/102394
- WO-A-03/082313
- WO-A-2004/080196
- WO-A-2004/082399
- US-A- 5 444 054
- GUERIN M ET AL: "Haematococcus astaxanthin: applications for human health and nutrition" TRENDS IN BIOTECHNOLOGY, ELSEVIER PUBLICATIONS, CAMBRIDGE, GB, vol. 21, no. 5, May 2003 (2003-05), pages 210-216, XP004422156 ISSN: 0167-7799
- WANG X ET AL: "Astaxanthin-rich algal meal and vitamin C inhibit Helicobacter pylori infection in BALB/cA mice" ANTIMICROBIAL AGENTS AND CHEMOTHERAPY 2000 UNITED STATES, vol. 44, no. 9, 2000, pages 2452-2457, XP008039976 ISSN: 0066-4804
- MURTHY R ET AL: "Omega-3 fatty acids as anti-inflammatory agents: A classical group of nutraceuticals" JOURNAL OF NUTRACEUTICALS, FUNCTIONAL AND MEDICAL FOODS 1999 UNITED STATES, vol. 2, no. 1, 1999, pages 53-72, XP008039978 ISSN: 1089-4179

## Description

The invention relates to a composition comprising a source of docosahexaenoic acid (DHA), and astaxanthin, and other nutrients for prophylactic and/or therapeutic use in the healing of trauma- and stress-induced inflammatory conditions.

### Background to the invention

Inflammation is a complex stereotypical reaction of the body responding to damage of its cells and vascularised tissues. The damaged sites are susceptible to infiltration by a multitude of pathogens including viruses, bacteria, fungi, and protozoan and metazoan parasites, as well as cancerous cells and other harmful agents and so the animal defends itself by initiating an inflammatory reaction at the damaged site.

The inflammatory reaction is phylogenetically and ontogenetically the oldest defence mechanism and both the innate and adaptive immune systems in vertebrates are triggered to destroy the infectious agent(s). When a tissue has been traumatised, for example, by injury or surgery, and is thus susceptible to infection, three key steps in the inflammatory response are initiated; (1) vasodilation, which enables an increased blood supply to the traumatised tissue; (2), increased capillary permeability caused by retraction of the endothelial cells allowing soluble mediators of immunity to reach the site of inflammation; and (3) migration of leukocytes (neutrophils; monocytes and lymphocytes) out of the capillaries into the surrounding tissues.

The development of inflammatory reactions is controlled in part by pro-inflammatory cytokines (e.g. interleukin-1, tumour necrosis factor alpha); by lipid mediators released from different cells (e.g. prostaglandin's and leukotrienes); by cell-derived vasoactive mediators released from mast cells, basophils and platelets (e.g. arachidonic acid metabolites; platelet activating factors amines: serotonin, histamine; endothelins) and by plasma-derived vasoactive mediators (e.g. kinins and components of the complement, coagulation and fibrinolytic cascades).

Chronic inflammation is an inflammatory response of prolonged duration - weeks, months, or even indefinitely - whose extended time course is provoked by the persistence of the causative stimulus to inflammation within the tissue. The inflammatory process inevitably causes tissue damage and is accompanied by misdirected attempts at simultaneous healing and repair. The exact nature, extent and time course of chronic inflammation is variable, and depends on a balance between the causative agent and the attempts of the body to remove it.

Chronic inflammation may develop either as a progression from acute inflammation if the original stimulus persists, or after repeated episodes of acute inflammation or *de novo* if the causative agent produces only a mild acute response.

Aetiological agents producing chronic inflammation include, but are not limited to: infectious organisms that can avoid or resist host defences and so persist in the tissue for a prolonged period; infectious organisms that are not innately resistant but persist in damaged regions where they are protected from host defences; irritant non-living foreign material that cannot be removed by enzymatic breakdown or phagocytosis; or where the stimuli is a "normal" tissue component, causing an auto-immune disease.

There is a vast array of diseases exhibiting a chronic inflammatory component. These include but are not limited to: inflammatory joint diseases (e.g., rheumatoid arthritis, osteoarthritis, polyarthritis and gout), chronic inflammatory connective tissue diseases (e.g., lupus erythematosus, scleroderma, Sjorgen's syndrome, poly- and dermatomyositis, vasculitis, mixed connective tissue disease (MCTD), tendonitis, synovitis, bacterial endocarditis, osteomyelitis and psoriasis), chronic inflammatory lung diseases (e.g., chronic respiratory disease, pneumonia, fibrosing alveolitis, chronic bronchitis,chronic obstructive pulmonary disease (COPD), bronchiectasis, emphysema, silicosis and other pneumoconiosis and tuberculosis), chronic inflammatory bowel and gastro-intestinal tract inflammatory diseases (e.g., ulcerative colitis and Crohn's disease), chronic neural inflammatory diseases (e.g., chronic inflammatory demyelinating polyradiculoneuropathy, chronic inflammatory demyelinating polyneuropathy, multiple sclerosis, Guillan-Barre Syndrome and myasthemia gravis), other inflammatory diseases (e.g., mastitis, laminitis, laryngitis, chronic cholecystitis, Hashimoto's thyroiditis, inflammatory breast disease); chronic inflammation caused by an implanted foreign body in a wound; and acute inflammatory tissue damage due to muscle damage after eccentric exercise (e.g., delayed onset muscle soreness - DOMS).

The usual mode of treatment for chronic inflammatory conditions is by administration of non-steroidal anti-inflammatory drugs (NSAID's) such as Diclofenac, Ibuprofen, Aspirin, Phenylbutazone, Indomethacin , Naproxen and Piroxicam. Although NSAID's can be effective, they are known to be associated with a number of side effects and adverse reactions. These may include gastro-intestinal problems such as dyspepsia, ulceration and haemorrhage, sleepiness, nausea or vomiting, constipation, allergic reactions and occasionally hepatoxicity. Frequently the margin between effective dose and toxic dose is quite small (i.e., 2-3 -fold). In spite of these side effects, the use of NSAID's as anti-inflammatory agents is standard practice in human medicine and veterinary medicine. However, within veterinary medicine there is an increasing concern about their use in food animals because of the potential for accumulation of drugs such as phenylbutazone within the food chain.

It is the purpose of this invention to provide a natural alternative to anti-inflammatory drugs widely used to treat chronic inflammatory conditions of terrestrial animals including humans. The use of such an alternative will be safe and without side-effects or risks to the environment.

DHA is an omega-3 fatty acid and is the most abundant long chain polyunsaturated fatty acid (PUFA) in the grey matter of the brain and other neurological tissues. Omega-3 PUFAs, particularly eicosapentaenoic acid (EPA) are known to be beneficial in reducing incidence of coronary heart disease (Lands, Fish and Human Health 1986 Academic Press). The anti-inflammatory properties of omega-3 PUFAs are thought to be provided by their ability to replace arachidonic (ARA) acid in immune cells membranes. ARA, an omega-6 PUFA with 20 carbon atoms and 4 double bonds (C20:4), is the biochemical precursor for the production of 2-series prostaglandins and 4-series leukotrienes associated with a range of pro-inflammatory molecules and mediators and can therefore impact pathogenesis of inflammatory diseases. (P. Calder "n-3 Fatty Acids & Health Conference (December 1999) British Nutrition Foundation). EPA, an omega-3 PUFA with 30 carbon atoms and 5 double bonds (C20:5) is the biochemical precursor for the production of 3-series prostaglandins and 2-series leukotrienes which are anti-inflammatory molecules. Thus, the balance of EPA and ARA is thought to significantly affect the balance of pro- and anti-inflamatory eicosanoid mediators. DHA, an omega-3 PUFA with 22 carbon atoms and 6 double bonds (C22:6) does not form eicosanoids (i.e., 20C prostaglandins or leukotrienes). Omega-3 fatty acids have a long history of use in animal feeding via use of cod liver oil, linseed and flax oil.

A metabolic pathway exists in mammals for the biosynthesis of DHA, but this is bio-energetically unfavourable (Crawford, P. AOCS, Short Course in Polyunsaturated Fatty Acids and Eicosanoids, pp270-295 (1987)). The metabolism of omega-3 fatty acids is not well understood, thus precise clinical dosage rates and efficacy remain unknown. Mammals are thought to obtain most of their DHA from dietary sources.

Omega-3 and omega-6 fatty acids are found in cold-water marine fish; and fish oils are the primary commercial source of these fatty acids. Environmental pollution of fish introduces toxic factors such as dioxins and PCB's to the oils recovered from fish, which if ingested may adversely affect the health of all animals and may remain as residues in food animals rendering them problematic for human consumption.

Marine microorganisms are known to contain DHA, in particular dinoflagellates (Harrington et al "The Polyunsaturated Fatty Acids of Marine Dinoflagellates" J. Protozoal, 17:213-219 (1970)). Successful cultivation of these in commercial conditions is achievable (U.S 5,407,957). In adequate presence of Vitamin E animals can consume up to 2% of their diet as DHA when using fish oil, but higher levels result in malodorous products.

Astaxanthin is a carotenoid known to be partially degraded in the gastro-intestinal tract by oxidation. The presence of vitamins A, C, selenium, manganese, zinc and copper are known to alleviate this effect. Certain microorganisms including but not limited to algae and yeast are know to be prolific producers of astaxanthin. Both forms of algae, and yeast contain adequate combinations of the above elements to counteract the oxidative effect of digestive oxidation to both the lipids and the astaxanthin therein.

Other marine organisms, including but not limited to zooplankton, crustaceans, molluscs, and vertebrates, are also known to contain high levels of the carotenoid astaxanthin. It has been shown that in fish and crustaceans, astaxanthin is essential for growth and plays a vitamin-like role. Astaxanthin also appears to have some beneficial effects on mammals. Astaxanthin is an active ingredient in several patented medications for mammals. In an anti-stress formulation, it is claimed to enhance the effect of anti-stress agents administered to farm animals and household pets to minimise weight loss and reduced immunity due to crowding, extreme temperatures and other sudden environmental changes (U.S 5,937,790).

Esterified astaxanthin from the alga *Haematococcus pluvialis* is the preferred form in several oral prophylactic and therapeutic formulations for muscular dysfunction such as exertional rhabdomyolysis (also known as exertional myopathy, tying-up syndrome, azoturia, or Monday morning sickness) in horses (WO 99/11251), as well as for mastitis (mammary inflammation) in dairy cows (WO 99/30701), and for mammalian gastrointestinal tract inflammation due to infections by *Helicobacter* sp. bacteria (WO 98/37874).

The use of yeast in animal feed has a long and well-documented history. Recent changes to European law (EC Directive 87/153/EEC and associated reports) specifically in respect to the ability of a gastro-intestinal tract to resist overgrowth by any one component or strain is now active. Whilst the mode of action is not documented, it is thought that there are similarities between the action of the rumen and the cecal fermentation of mammals that rely on bacterial fermentation resulting in production of lactic acid. (Martin, S.A. and Nisbet, D.J., "Effect of direct-fed microbials on rumen microbial fermentation" J. Dairy Sci. 75:1736 (1992))

Recent research and meta-analysis shows yeast to improve digestion and availability of nutrients when nutritional demands are high. Positive effects on the efficacy of immune systems to increase macrophage activity against *E. coli* and *Salmonella typhirium* have been shown. (Hatcher G. E., Lambretch R.S., "Augmentation of macrophage phagocytic activity by cell-free extracts of selected lactic acid producing bacteria" J. Dairy Sci. 76:2485 (1993) and Schiffrin, E.J. et al, "Immunomodulation of human blood cells following the ingestion of lactic acid bacteria J. Dairy Sci 78: 491 (1995)).

EP 0 699 437 describes pharmaceutical preparations containing omega-3 fatty acids in combination with antioxidant/reducing vitamins or provitamins. WO 02/102,394 describes the administration of a therapeutically effective amount of Krill and/or marine oil to a patient in a method of treatment and/or prevention of cardiovascular disease, rheumatoid arthritis, skin cancer, premenstrual syndrome, diabetes and transdermal transport enhancement. US 5,444,054 describes a method of improving the nutritional status and reversing the characteristic diarrhea and inflammatory condition in a mammalian creature having ulcerative colitis or inflammation of the colon which contains in combination an oil blend which contains eicosapentaenoic acid (20:5n3) and/or docosahexaenoic acid (22:6n3), and a source of indigestible carbohydrate which is metabolized to short chain fatty acids by microorganisms present in the human colon. WO98/37874 describes an oral preparation, comprising at least one type of xanthophylles, for the prophylactic and/or therapeutic treatment of inflammation in the mucous membrane of mammalian gastrointestinal tract (especially the human stomach) caused by Helicobacter sp. (especially H. pylori) infection. WO01/24787 describes the use of at least one type of xanthophylls for the production of a medicament for suppression of excessive Th1 cell mediated immune responses and stimulation of Th2 cell mediated immune responses in a patient during ongoing infection and/or inflammation. Guerin et al, Trends Biotechnol. 2003 May;21(5):210-6, describes the important applications of the carotenoid pigment astaxanthin. Wang et al, Antimicrob Agents Chemother. 2000 Sep;44(9):2452-7, describes the affect of astaxanthin and vitamin C on *H.pylori* infection. Murthy and Murthy, Journal of Nutraceuticals, Functional & Medical Food, 1999:2(1): 53-72, describes long chain omega-3 fatty acids and their nutraceutical use.

An object of the present invention is to provide a dietary supplement to an animal, including humans, that will provide a protective benefit to animals in high stress environments such as competition or transportation, and/or to be used therapeutically to further enhance the healing of trauma and stress-induced inflammatory conditions.

A further objective of this invention is to provide a natural alternative to anti-inflammatory drugs currently used in traditional veterinary and human medicine.

We have found that a combination of docosahexaenoic acid and astaxanthin provides an unexpectedly beneficial effect in reducing the negative effects of inflammatory processes induced by trauma or stress, and further that these materials can be provided to an animal, including humans, in a natural and bioavailable form.

### Summary of the Invention

In a first aspect the invention provides use of a composition comprising docosahexaenoic acid or an ester thereof, wherein the ester of docosahexaenoic acid is selected from the group consisting of: a triglyceride, diglyceride, monoglyceride, phospholipids, glycolipid, sphingolipid or sulpholipid and at least one carotenoid, wherein said carotenoid is astaxanthin and is provided from an algal or yeast source, in the manufacture of a medicament for the treatment of a trauma or stress-induced inflammatory condition.

Preferably, said docosahexaenoic acid is provided as an algae or fraction thereof, more preferably said algae is selected from the group consisting of: *Crypthecodinium; Phaedactylum; Isochrysis; Schizochytrium; Thaustochytrium;* or *Ulkenia.*

Preferably said docosahexaenoic acid is provided as a fish oil or fraction thereof Preferably said yeast source is *Haematococcus* or *Phaffia.*

Preferably said docosahexaenoic acid is provided to an animal at a dose of between 0.05 and 500 mg/kg body weight and said carotenoid is provided at a dose of between 0.5 and 5,000 ug/kg body weight. Alternatively, said docosahexaenoic acid is provided to an animal at a dose of between 0.5 and 15 mg/kg body weight and said carotenoid is provided at a dose of between 1.5 and 150 ug/kg body weight. Alternatively said docosahexaenoic acid is provided to an animal at a dose of between 1 and 3 mg/kg body weight and said carotenoid is provided at a dose of between 7.5 and 22.5 ug/kg body weight.

Preferably said composition further comprises yeast.

Preferably said composition further comprises an anti-inflammatory agent, more preferably said further anti-inflammatory agent is selected from the group consisting of: vitamin C, vitamin E, lycopene, β-carotene, lutein, organic selenium, α-lipoic acid, methylsulfonylmethane, glutathione, taurine, glycine, glutamine, arginine, cysteine, methionine, S-adenosylmethionine, nucleotides, nucleic acids, curcumin, green tea extract, green-lipped mussel extract (Perna canaliculus), or standardised herbal extracts such as *Phyllanthus amarus, Fructus Schisandra ,* Chamomile, Blackcurrant leaf, Devil's claw.

In a further aspect the invention provides a composition comprising an algal source of docosahexaenoic acid and a yeast source of astaxanthin for use as a pharmaceutical.

Preferably the algae is selected from the group consisting of: *Crypthecodinium; Phaedactylum; Isochrysis; Schizochytrium_{;} Thaustochytrium;* or *Ulkenia.*

Preferanbly the yeast source of astaxanthin is *Haematococcus* or *Phaffia.*

Preferably the composition further comprises inactivated brewers yeast.

Preferably the composition further comprises an anti-inflammatory agent selected from the group consisting of: vitamin C, vitamin E, lycopene, β-carotene, lutein , organic selenium, α-lipoic acid, methylsulfonylmethane, glutathione, taurine, glycine, glutamine, arginine, cysteine, methionine, S-adenosylmethionine, nucleotides, nucleic acids, curcumin, green tea extract, green-lipped mussel extract (Perna canaliculus), or standardised herbal extracts such as Phyllanthus amarus, Fructus Schisandra, Chamomile, Blacurrant leaf, Devil's claw.

Preferably the pharmaceutical is a neutraceutical.

According to a further aspect of the invention there is provided a composition according to any previous aspect or embodiment for use as a nutraceutical.

When administered, compositions of the present invention are administered in physiologically acceptable preparations. Such preparations may routinely contain physiologically acceptable concentrations of salt, buffering agents, preservatives, compatible carriers and optionally other therapeutic agents.

The compositions of the invention can be administered by any conventional route, including, but not limited to, injection, or gradual infusion over time. The administration may, for example, be oral, intravenous, intraperitoneal, intramuscular, intracavity, subcutaneous, or transdermal. Preferably said compositions are administered orally in the feed or as a feed supplement. Alternatively, the compositions can be provided in the water or as a tonic.

The compositions of the invention are administered in effective amounts. An "effective amount" is that amount of a composition that alone, or together with further doses, produces the desired response. In the case of treating a particular disease, such as arthritis, the desired response is inhibiting the progression of the disease. This may involve only slowing the progression of the disease temporarily, although more preferably, it involves halting the progression of the disease permanently. This can be monitored by routine methods.

Such amounts will depend, of course, on the particular condition being treated, the severity of the condition, the individual patient parameters including age, physical condition, size and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. The compositions used in the foregoing methods preferably are sterile and contain an effective amount of the active agents for producing the desired response in a unit of weight or volume suitable for administration to a patient.

In general, the active agent DHA is formulated and administered in doses between 0.05-500mg/kg body weight, preferably between 0.5-15 mg/kg body weight, and most preferably between 1-3mg /kg body weight. The active agent astaxanthin is formulated and administered in doses between 0.0005mg-5mg/kg body weight, preferably between 0.0015-0.15mg/kg body weight, and most preferably between 0.0075-0.0225mg/kg body weight according to any standard procedure in the art.

Compositions may be combined, if desired, with a physiologically-acceptable carrier. The term "physiologically-acceptable carrier" as used herein means one or more compatible solid or liquid fillers, diluents or encapsulating substances that are suitable for administration into a human or animal. The term "carrier" denotes an organic or inorganic ingredient, natural or synthetic, with which the active ingredient is combined to facilitate the application. The compositions may contain suitable buffering agents.

The compositions may conveniently be presented in unit dosage form and may be prepared by any of the methods well-known in the food industry for the preparation of food and food supplements, or by methods known to the pharmaceutical industry. Methods known to those skilled in the art of food manufacturing include but are not limited to dry-blending of active agents and other ingredients in powder form, spray-drying of emulsions containing all components or the use of extrusion technologies to form pellets or granules.

Pharmaceutical methods include the step of bringing the active agent into association with a carrier which constitutes one or more accessory ingredients. In general, the compositions are prepared by uniformly and intimately bringing the active compound into association with a liquid carrier, a finely divided solid carrier, or both, and then, if necessary, shaping the product. This can then be either administered directly to the animal or added to food.

Compositions suitable for oral administration may be presented as discrete units, such as capsules, tablets, lozenges, each containing a predetermined amount of the active agent. Other compositions include suspensions in aqueous liquids or non-aqueous liquids such as a syrup, elixir, tonic, or an emulsion.

In a still further aspect the invention provides a feed comprising a docosahexaenoic acid or an ester thereof, wherein the ester of docosahexaenoic acid is selected from the group consisting of: a triglyceride, diglyceride, monoglyceride, phospholipids, glycolipid, sphingolipid or sulpholipid and at least one carotenoid, characterized in that said carotenoid is astaxanthin and is provided from a microbial source.

Preferably the feed further comprises grass meal.

In a still further aspect the invention provides use of a composition in accordance with a first aspect of the invention wherein the medicament is a neutraceutical.

According to a further aspect of the disclosure there is provided the use of a composition according to the invention for the manufacture of a nutraceutical for use in the treatment of inflammatory conditions.

In a preferred embodiment of the disclosure said inflammatory condition is selected from the group consisting of, but not limited to inflammatory joint diseases (e.g. rheumatoid arthritis, osteoarthritis, polyarthritis and gout); chronic inflammatory connective tissue diseases (e.g. lupus erythematosus, scleroderma, Sjorgen's Syndrome, poly- and dermatomyositis, vasculitis); mixed connective tissue disease (MCTD) (e.g. tendonitis, synovitis, bacterial endocarditis, osteomyelitis and psoriasis); chronic inflammatory lung diseases (e.g. chronic respiratory disease, pneumonia, fibrosing alveolitis, chronic bronchitis, chronic obstructive pulmonary disease (COPD), bronchiectasis, emphysema, silicosis and other pneumoconiosis and tuberculosis); chronic inflammatory bowel and gastro-intestinal tract inflammatory diseases (e.g. ulcerative colitis and Crohn's disease); chronic neural inflammatory diseases (e.g. chronic inflammatory demyelinating polyradiculoneuropathy, chronic inflammatory demyelinating polyneuropathy, multiple sclerosis, Guillan-Barre Syndrome and myasthemia gravis); and other inflammatory diseases including, mastitis, laminitis, laryngitis, chronic cholecystitis, Hashimoto's thyroiditis, inflammatory breast disease; chronic inflammation caused by an implanted foreign body in a wound; and acute inflammatory tissue damage due to muscle damage after eccentric exercise (e.g., delayed onset muscle soreness - DOMS).

According to a further aspect of the disclosure there is provided a food stuff wherein said food stuff comprises a composition according to any previous aspect of embodiment.

According to a further aspect of the disclosure there is provided a method to treat an animal suffering from an inflammatory condition or disease comprising administering to said animal an effective amount of at least one long chain polyunsaturated fatty acid and at least one carotenoid.

In a preferred method of the disclosure said long chain fatty acid is a free fatty acid, or an ester thereof.

In a further preferred method of the disclosure said long chain fatty acid is selected from the group consisting of a triglyceride, diglyceride, monoglyceride, phospholipids, glycolipid, sphingolipid or sulpholipid.

In a further preferred method of the disclosure said long chain fatty acid is docosahexaenoic acid.

In a yet further preferred method of the disclosure said carotenoid is astaxanthin.

In a further preferred method of the invention said animal is administered a further anti-inflammatory agent.

In a preferred method of the disclosure said disease for condition is selected from the group consisting of but not limited to: inflammatory joint diseases (e.g. rheumatoid arthritis, osteoarthritis, palyarthritis and gout); chronic inflammatory connective tissue diseases (e.g. lupus erythematosus, scleroderma, Sjorgen's syndrome, poly- and dermatomyositis, vasculitis); mixed connective tissue disease (MCTD)(e.g. tendonitis, synovitis, bacterial endocarditis, osteomyelitis and psoriasis); chronic inflammatory lung diseases (e.g. chronic respiratory disease, pneumonia, fibrosing alveolitis, chronic bronchitis, chronic obstructive pulmonary disease (COPD), bronchiectasis, emphysema, silicosis and other pneumoconiosis and tuberculosis); chronic inflammatory bowel and gastro-intestinal tract inflammatory diseases (e.g. ulcerative colitis and Crohn's disease); chronic neural inflammatory diseases (e.g. chronic inflammatory demyelinating polyradiculoneuropathy, chronic inflammatory demyelinating polyneuropathy, multiple sclerosis, Guillan-Barre Syndrome and myasthemia gravis); and other inflammatory diseases including mastitis, laminitis, laryngitis, chronic cholecystitis, Hashimoto's thyroiditis, inflammatory breast disease; chronic inflammation caused by an implanted foreign body in a wound; and acute inflammatory tissue damage due to muscle damage after eccentric exercise (e.g., delayed onset muscle soreness - DOMS).

In a preferred method of the disclosure said animal is a terrestrial animal

In a further preferred method of the disclosure said animal is a companion or performance animal.

In a further preferred method of the disclosure said animal is selected from the group consisting of: human, horse, cow; sheep; goat; Ilama, camel, mink; pig dog; cat; hamster; mouse; rabbit; pot bellied pig; rat, gerbil, guinea pig.

In a preferred method of the disclosure said animal is a horse.

In a further preferred method of the disclosure said animal is a human.

An embodiment of the invention will now be described by example only and with reference to the following materials, methods and examples.

### Materials and Methods

### Sources of DHA and astaxanthin:

DHA can be found in oils extracted from marine animals and organisms, including algae. Suitable commercial sources of DHA include, but are not limited to algae such as *Crypthecodinium; Phaedactylum; Isochrysis; Schizochytrium; Thaustochytrium;* or *Ulkenia,* or purified, or semipurified lipid products from these species.

Alternatively DHA can be provided by commercially available marine oils which typically contain levels between 15% and 25% DHA and between 5% and 15% EPA (w/w). Suitable marine oils include, but are not limited to: crude or processed fish oil, krill oil, squid oil, or refining or processing coproducts from the manufacture of these oils.

Suitable sources of commercially available astaxanthin include, but are not limited to: the dried algae product *Haematococcus pluvialis* (Cyanotech Corp, USA), dehydrated yeast product *Phaffia rhodozyma* (Igene Corp, USA). Alternatively the commercially available synthetic form of astaxanthin may be used (Roche, Switzerland; BASF, Germany).

### Manufacture of pellets for animal feeds :

Ingredients as described in the examples below (formulas 1-18) are dry-blended together with oatmeal, grass meal , calcium carbonate, liquid oat oil and a suitable pellet binder. The mixture is processed using cool extrusion technology as routinely used by those skilled in the art of food manufacture.

Most preferred levels of inclusion of fomulas 1-18 typically range from 5-40%

### Manufacture of soft gel capsules suitable for human consumption:

Suitable inner filling components are described by, but not limited to , formulas 19-21 and formulas 25-26. A liquid premix , prepared with optional use of emulsifiers and stabilising agents comprises about 70% by weight of the capsule. The outer shell of the capsule (approx. 30% total capsule weight) comprises predominantly gelatin or a vegetable gum alternative as well as glycerol and flavouring/colouring components.

### TABLE 1. EXAMPLES OF SUITABLE FORMULAE FOR PRODUCT MANUFACTURE

### Formula 1.

| **Ingredient** | % |
|---|---|
| Crypthecodinium cohnii (dried biomass) | 60 |
| Haematococcus (dried biomass) | 5 |
| Inactivated Brewers dried yeast | 35 |

### Formula 2.

| **Ingredient** | % |
|---|---|
| Crypthecodinium cohnii (dried biomass) | 40.0 |
| Haematococcus (dried biomass) | 3.4 |
| Inactivated Brewers dried yeast | 23.3 |
| Grass Meal | 33.3 |

### Formula 3.

| **ingredient** | % |
|---|---|
| Schizochytrium sp. (dried biomass) | 60 |
| Haematococcus (dried biomass) | 5 |
| Inactivated Brewers dried yeast | 35 |

### Formula 4.

| **Ingredient** | % |
|---|---|
| Schizochytrium sp.(dried biomass) | 40.0 |
| Haematococcus (dried biomass) | 3.4 |
| Inactivated Brewers dried yeast | 23.3 |
| Grass Meal | 33.3 |

### Formula 5

| **Ingredient** | % |
|---|---|
| Crypthecodinium cohnii (dried biomass) | 60.0 |
| Astaxanthin (Phaffia) | 5 |
| Inactivated Brewers dried yeast | 35 |

### Formula 6.

| **Ingredient** | % |
|---|---|
| Crypthecodinium cohnii (dried biomass) | 60.0 |
| Astaxanthin (synthetic) | 5 |
| Inactivated Brewers dried yeast | 35 |

### Formula 7.

| **Ingredient** | % |
|---|---|
| Crypthecodinium cohnii (dried biomass) | 40.0 |
| Astaxanthin (Phaffia) | 3.4 |
| Inactivated Brewers dried yeast | 23.3 |
| Grass Meal | 33.3 |

### Formula 8.

| **Ingredient** | % |
|---|---|
| Crypthecodinium cohnii (dried biomass) | 40.0 |
| Astaxanthin (synthetic) | 3.4 |
| Inactivated Brewers dried yeast | 23.3 |
| Grass Meal | 33.3 |

### Formula 9.

| **Ingredient** | % |
|---|---|
| Schizochytrium sp. (dried biomass) | 60 |
| Astaxanthin (Phaffia) | 5 |
| Inactivated Brewers dried yeast | 35 |

### Formula 10.

| **Ingredient** | |
|---|---|
| Schizochytrium sp. (dried biomass) | 60 |
| Astaxanthin (synthetic) | 0.05 |
| Inactivated Brewers dried yeast | 35 |
| Grass Meal | 4.95 |

### Formula 11.

| **Ingredient** | % |
|---|---|
| Schizochytrium sp.(dried biomass) | 40.0 |
| Astaxanthin (Phaffia) | 3.4 |
| Inactivated Brewers dried yeast | 23.3 |
| Grass Meal | 33.3 |

### Formula 12.

| **Ingredient** | % |
|---|---|
| Schizochytrium sp.(dried biomass) | 40.0 |
| Astaxanthin (synthetic) | 0.05 |
| Inactivated Brewers dried yeast | 23.3 |
| Grass Meal | 36.65 |

### Formula 13.

| **Ingredient** | % |
|---|---|
| Fish oil (microencapsulated) | 75 |
| Haematococcus (dried biomass) | 3 |
| Inactivated Brewers dried yeast | 22 |

### Formula 14.

| **Ingredient** | % |
|---|---|
| Fish oil (microencapsulated) | 75 |
| Pfaffia (astaxanthin) | 3 |
| Inactivated Brewers dried yeast | 22 |

### Formula 15.

| **Ingredient** | % |
|---|---|
| Fish oil (microencapsulated) | 75 |
| Astaxanthin (synthetic) | 0.05 |
| Inactivated Brewers dried yeast | 24.95 |

### Formula 16.

| **Ingredient** | % |
|---|---|
| Fish oil (microencapsulated) | 75 |
| Haematococcus (dried biomass) | 3 |
| Inactivated Brewers dried yeast | 20 |
| Grass Meal | 2 |

### Formula 17.

| **Ingredient** | % |
|---|---|
| Fish oil (microencapsulated) | 75 |
| Astaxanthin (Phaffia) 3 | 3 |
| Inactivated Brewers dried yeast | 20 |
| Grass Meal | 2 |

### Formula 18,

| **Ingredient** | % |
|---|---|
| Fish oil (microencapsulated) | 75 |
| Astaxanthin (synthetic) | 3 |
| Inactivated Brewers dried yeast | 20 |
| Grass Meal | 2 |

### Formula 19.

| **Ingredient** | % |
|---|---|
| DHA algal oil (DHASCO)® | 15 |
| Haematococcus (dried biomass) | 15 |
| Inactivated Brewers dried yeast | 35 |
| other ingredients | To 100 |

### Formula 20.

| **Ingredient** | % |
|---|---|
| DHA algal (DHASCO)® | 15 |
| Astaxanthin (Phaffia) | 3 |
| Inactivated Brewers dried yeast | 35 |
| Other ingredients | To 100 |

### Formula 21.

| **Ingredient** | % |
|---|---|
| DHA algal oil (DHASCO)® | 15 |
| Astaxanthin (synthetic) | 0.045 |
| Inactivated Brewers dried yeast | 35 |
| Other ingredients | To 100 |

### Formula 22.

| **Ingredient** | % |
|---|---|
| DHA algal oil (DHASCO)® | 15 |
| Haematococcus (dried biomass) | 3.4 |
| Inactivated Brewers dried yeast | 23.3 |
| Grass Meal | 25.3 |
| Other ingredients | To 100 |

### Formula 23.

| **Ingredient** | % |
|---|---|
| DHA algal oil (DHASCO)® | 15 |
| Astaxanthin (Phaffia) | 3.4 |
| Inactivated Brewers dried yeast | 23.3 |
| Grass Meal | 25.3 |
| Other ingredients | To 100 |

### Formula 24.

| **Ingredient** | % |
|---|---|
| DHA algal oil (DHASCO)® | 15 |
| Astaxanthin (synthetic) | 0.1 |
| Inactivated Brewers dried yeast | 23.3 |
| Grass Meal | 25.3 |
| Other ingredients | To 100 |

### Formula 25.

| **Ingredient** | % |
|---|---|
| DHA (Any source) | 20 |
| Astaxanthin (any source) | 0.15 |
| α-Lipoic acid | 0.2 |
| Natural flavours | 0.3 |
| Maltodextrin 20 DE | To 100 |

### Formula 26.

| **Ingredient** | % |
|---|---|
| DHA (Any source) | 20 |
| Astaxanthin (any source) | 0.15 |
| Methyl sulfonyl methane (any source) | 0.2 |
| Natural flavours | 0.3 |
| Maltodextrin 20 DE | To 100 |

### EXAMPLE 1

### Case Study 1

A 26-year-old, 15.2hh ¾ thoroughbred gelding had undergone metacarpal surgery on his off-fore knee 15 months prior to intervention. The animal was suffering osteoarthritis, general stiffness more deterioration in ability to movement when cold. Long-term soft tissue swelling at the site of the injury and surgery remained round knee joint decreasing possibility of flexion further. Oedema surrounding ligaments and tendons occurred upon inactivity (e.g. stabling)

Dietary intake had included a range of herbal supplements, but no chemical phenylbutazone or other anti-inflammatory substances; 95% forage based with 1kg per day cereal mix. Initial dose of 0.5g formula 1 per day was included in the diet, increasing over 14 days to 8g invention per day. Soft tissue swelling reduced above and below knee within 7 days (5mm decrease in circumference above knee and 4mm below knee), oedema reduced in hind lower limbs, stiffness on activity following rest was noticeably reduced. After 21 days general alertness and overall health was noticed to have improved, e.g. coat condition. Horse was more eager to canter in field and less prone to stumbling on off fore.

### Comparative EXAMPLE 2

### Case Study 2

The subject was a 23-year-old, 13.2hh cob mare exhibiting old age related stiffness, notably following work on hard ground and when weather was cold and wet. Clinical symptoms were reluctance to move quickly whether ridden or in-hand, stiffness when moving out of stable following period of inactivity, oedema in lower limbs, slight grumpy manner when being handled and reluctance to engage in spontaneous movement in field. General health was good. Diet was 95% forage based, with small amount of soaked sugar beet pulp per day. No drug therapy was used, but pony has previously received phenylbutazone for stiffness and swellings.

Initial dose of 5g formula 1 per day for 5 days showed dramatic improvement with eagerness to move both ridden, in-hand and when free. Energy levels increased with improvement in disposition when handled, pony started to jump out of field over 1 metre 10cm high fence which previously was impossible for her, stride length of hind limbs increased to allow hoof prints of front hooves to be covered by hind hooves. Dose reduced to 2.5g per day, improvements still noticeable and oedema in lower limbs reduced. Maintenance dose of 2g invention per day showed no loss of activity. Comments on improvement in action, attitude and ability of pony noted by owner, farrier and instructor, none of whom were aware of the dietary changes made.

### EXAMPLE 3

### Case Study 3

Subject was a 11-year-old 12hh show pony suffering from laminitis, and not ridden due to chronic lameness, possibly due to muscle, shoulder injury sustained 22 months prior to test. No improvement when given phenylbutazone or other anti-inflammatory substances, difficult for farrier to work on as pony unable to move leg away from body at an angle; could not hold balance with foot off ground and was very stiff and sore for 2 to 5 days following attention from farrier, a result of the injury. 95% forage based diet with 1kg cereal inclusion per day, turn out in field but no exercise. Pony very stiff at all times, movement across uneven terrain difficult, not able to be ridden.

Initial dose of 2.5g formula 1 per day for 5 days showed dramatic improvement with pony much freer in action. Pony jumped out of field over 1 meter 10cm fencing, landing on hard ground and was still sound, even the next day. After 4 weeks farrier shod pony and used him as an example to train other farriers because of his history of laminitis. Pony's ability to move leg, shoulder and withstand repeated lifting of legs was totally unexpected. Maintenance dose of 2g per day was used thereafter.

### Comparative EXAMPLE 4

### Case Study 4

Subject was a 10-year-old King Charles Cavalier spaniel diagnosed with rheumatism in left hip, noticeable stiffness and inability to use hind limbs after exercise and worse in cold weather. Daily treatments with 0.5 g of formula 1 resulted in improvement in coat and ability to exercise without pain (lifting of leg, limping, stopping suddenly) within 4 days. Dose was dropped to 0.5g per day on alternate days after 10 days of initial treatment and the animal continued to improve.

### Comparative EXAMPLE 5

### Case Study 5

Subject was an 8 year old miniature Poodle, with general stiffness, unwillingness to jump on chairs, not using one hind limb when walking, notably stiff when getting up after rest, difficulty in using stairs of house and not playing with other dogs. Intervention with Formula 1 at a dose of 0.25g per day with food for 7 days showed marked improvement in dogs movement, ability to jump on chairs/laps, and speed of ascent and descent of stairs. Play with other dog was initiated and speed of game was increased. Level of dose was maintained with overall improvement in dog's quality of life seen and overall condition.

### EXAMPLE 6

### Case Study 6

Subject was a 12 year old event mare with long term recurrent check ligament injury and residual swelling. Injury would reoccur after return to work, when work rate and load increased to increase fitness in an intermittent pattern. Condition was manageable with phenylbutazone, but residual swelling was not altered by this regime. Long term prognosis was retirements from competition and use as light hack or brood mare only, as competition laws do not allow use of non-steroidal anti-inflammatory substances. Intervention with Formula 2 at 0.25g per day increasing to a maximum daily load of 1g per day showed swelling reduced, intermittent lameness ceased; mare returned to full work load and regained competitive fitness levels without recurrence of injury. Mare is now competing again in Eventing and other sports.

### Comparative EXAMPLE 7

### Case Study 7

Subject was a 4 year old 16.2hh Irish Sports Horse who developed a splint on near fore measuring 6mm diameter; showing some signs of lameness, heat in splint formation and swelling in surrounding tendon sheaths. Vet recommended rest, treatment with phenylbutazone and cold hosing for 8 to 16 weeks with return to light work over period of 4 months if all signs of swelling had gone.

Phenylbutazone was not a preferred choice by the owner, so this was substituted by intervention with formula 2 at a dose of 8g per day. By Day 3 of intervention residual swelling in tendon sheaths had decreased, some remaining. Size of splint had decreased by 2mm diameter, all heat in splint was gone. After Day 8 treatment was suspended, and within 24 hours heat returned to splint, size increased back to 8mm and continued to increase on Day 9. Treatment was resumed on day 10, by Day 14 splint was cold and size reducing again. Treatment ongoing for minimum 14 days to settle splint formation, reduce heat and associated swelling.

## Claims

1. Use of a composition comprising docosahexaenoic acid or an ester thereof, wherein the ester of docosahexaenoic acid is selected from the group consisting of: a triglyceride, diglyceride, monoglyceride, phospholipids, glycolipid, sphingolipid or sulpholipid and at least one carotenoid, wherein said carotenoid is astaxanthin and is provided from an algal or yeast source, in the manufacture of a medicament for the treatment of a trauma or stress-induced inflammatory condition.

2. The use of Claim 1 wherein said docosahexaenoic acid is provided as an algae or fraction thereof.

3. The use of Claim 2 wherein said algae is selected from the group consisting of *Crypthecodinium; Phaedactylum; Isochrysis; Schizochytrium; Thaustochytrium;* or *Ulkenia.*

4. The use of Claim 1 wherein said docosahexaenoic acid is provided as a fish oil or fraction thereof.

5. The use according to Claim 1 wherein said yeast source is *Haematococcus* or *Phaffia.*

6. The use according to any of Claims 1 to 5 wherein said docosahexaenoic acid is provided to an animal at a dose of between 0.05 and 500 mg/kg body weight and said carotenoid is provided at a dose of between 0.5 and 5,000 ug/kg body weight.

7. The use according to any of Claims 1 to 5 wherein said docosahexaenoic acid is provided to an animal at a dose of between 0.5 and 15 mg/kg body weight and said carotenoid is provided at a dose of between 1.5 and 150 ug/kg body weight,

8. The use according to any of Claims 1 to 5 wherein said docosahexaenoic acid is provided to an animal at a dose of between 1 and 3 mg/kg body weight and said carotenoid is provided at a dose of between 7.5 and 22.5 ug/kg body weight.

9. The use according to any of Claims 1 to 8 wherein said composition further comprises yeast.

10. The use according to any of Claims 1 to 9 wherein said composition further comprises an anti-inflammatory agent.

11. The use according to Claim 10 wherein said further anti-inflammatory agent is selected from the group consisting of: vitamin C, vitamin E, lycopene, β-carotene, lutein, organic selenium, α-lipoic acid, methylsulfonylmethane, glutathione, taurine, glycine, glutamine, arginine, cysteine, methionine, S-adenosylmethionine, nucleotides, nucleic acids, curcumin, green tea extract, green-lipped mussel extract (Perna canaliculus), or standardised herbal extracts such as *Phyllanthus amarus, Fructus Schisandra ,* Chamomile, Blackcurrant leaf, Devil's claw.

12. A composition comprising an algal source of docosahexaenoic acid and a yeast source of astaxanthin for use as a pharmaceutical.

13. The composition of Claim 12 wherein the algae is selected from the group consisting of: *Crypthecodinium; Phaedactylum; Isochrysis; Schizochytrium; Thaustochytrium;* or *Ulkenia.*

14. The composition of Claim 12 or 13 wherein the yeast source of astaxanthin is *Haematococcus* or *Phaffia.*

15. The composition of Claims 12 to 14 further comprising inactivated brewers yeast.

16. The composition of any of claims 12 to 15 further comprising an anti-inflammatory agent selected from the group consisting of vitamin C, vitamin E, lycopene, β-carotene, lutein , organic selenium, α-lipoic acid, methylsulfonylmethane, glutathione, taurine, glycine, glutamine, arginine, cysteine, methionine, S-adenosylmethionine, nucleotides, nucleic acids, curcumin, green tea extract, green-lipped mussel extract (Perna canaliculus), or standardised herbal extracts such as Phyllanthus amarus, Fructus Schisandra, Chamomile, Blacurrant leaf, Devil's claw.

17. A feed comprising a docosahexaenoic acid or an ester thereof, wherein the ester of docosahexaenoic acid is selected from the group consisting of: a triglyceride, diglyceride, monoglyceride, phospholipids, glycolipid, sphingolipid or sulpholipid and at least one carotenoid, **characterized in that** said carotenoid is astaxanthin and is provided from a microbial source.

18. The feed according to claim 17 further comprising grass meal.

19. Use according to any one of claims 1 to 11 wherein the medicament is a neutraceutical.

20. The composition according of any of claims 12 to 15 wherein the pharmaceutical is a neutraceutical.

## Patentansprüche

1. Die Verwendung einer Zusammensetzung umfassend Docosahexaensäure oder einen Ester davon, wobei der Ester der Docosahexaensäure ausgewählt ist aus der Gruppe bestehend aus: einem Triglycerid, Diglycerid, Monoglycerid, Phospholipid, Glycolipid, Sphingolipid oder Sulpholipid und mindestens einem Carotenoid, wobei das Carotenoid aus Algen- oder Hefequelle gewonnenes Astaxanthin ist, zur Herstellung eines Medikaments zur Behandlung eines Traumas oder einer stressinduzierten entzündlichen Kondition.

2. Die Verwendung von Anspruch 1, wobei die besagte Docosahexaensäure als Alge oder als Teil einer Alge zur Verfügung gestellt wird.

3. Die Verwendung von Anspruch 2, wobei die besagte Alge ausgewählt ist aus der Gruppe bestehend aus: *Crypthecodinium; Phaedactylum; Isochrysis; Schizochytrium; Thaustochytrium;* oder *Ulkenia.*

4. Die Verwendung von Anspruch 1, wobei die besagte Docosahexaensäure als Fischöl oder Teil davon zur Verfügung gestellt wird.

5. Die Verwendung gemäß Anspruch 1, wobei die besagte Hefequelle *Haematococcus* oder *Phaffia* ist.

6. Die Verwendung gemäß einem der Ansprüche 1 bis 5, wobei die besagte Docosahexaensäure einem Tier in einer Dosis von zwischen 0,05 und 500 mg/kg Körpergewicht zur Verfügung gestellt wird, und das besagte Carotinoid in einer Dosis von zwischen 0,5 und 5,000 µg/kg Körpergewicht zur Verfügung gestellt wird.

7. Die Verwendung gemäß einem der Ansprüche 1 bis 5, wobei die besagte Docosahexaensäure einem Tier in einer Dosis von zwischen 0,5 und 15 mg/kg Körpergewicht zur Verfügung gestellt wird, und das besagte Carotinoid in einer Dosis von zwischen 1,5 und 150 µg/kg Körpergewicht zur Verfügung gestellt wird.

8. Die Verwendung gemäß einem der Ansprüche 1 bis 5, wobei die besagte Docosahexaensäure einem Tier in einer Dosis von zwischen 1 und 3 mg/kg Körpergewicht zur Verfügung gestellt wird, und das besagte Carotinoid in einer Dosis von zwischen 7,5 und 22,5 µg/kg Körpergewicht zur Verfügung gestellt wird.

9. Die Verwendung gemäß einem der Ansprüche 1 bis 8, wobei die besagte Zusammensetzung außerdem Hefe enthält.

10. Die Verwendung gemäß einem der Ansprüche 1 bis 9, wobei die besagte Zusammensetzung außerdem einen anti-inflammatorischen Wirkstoff enthält.

11. Die Verwendung gemäß Anspruch 10, wobei der besagte weitere anti-inflammatorische Wirkstoff ausgewählt ist aus der Gruppe bestehend aus: Vitamin C, Vitamin E, Lycopen, β-Carotin, Lutein, organisches Selen, α-Liponsäure, Methylsulfonlymethan, Glutathion, Taurin, Glycin, Glutamin, Arginin, Cystein, Methionin, S-Adenosylmethionin, Nukleotiden, Nukleinsäuren, Curcumin, Grünteeextrakt, Grünschalmuschelextrakt (*Perna canaliculus*)*,* oder standardisierten Kräuterextrakten wie *Phyllanthus amarus, Fructu,r schisandra,* Kamille, Schwarze Johannisbeere, Teufelskralle.

12. Eine Zusammensetzung umfassend eine Algenquelle für Docosahexaensäure und eine Hefequelle für Astaxanthin zur Verwendung als ein Pharmazeutikum.

13. Die Zusammensetzung von Anspruch 12, wobei die Alge ausgewählt ist aus der Gruppe bestehend aus: *Crypthecodinium; Phaedactylum; Isochrysis, Schizochytrium; Thaustochytrium;* oder *Ulkenia.*

14. Die Zusammensetzung gemäß Anspruch 12 oder 13, wobei die Hefequelle für Astaxanthin *Haematococcus* oder *Phaffia* ist.

15. Die Zusammensetzung der Ansprüche 12 bis 14, weiter umfassend inaktivierte Bierhefe.

16. Die Zusammensetzung gemäß einem der Ansprüche 12 bis 15, weiter umfassend einen anti-inflammatorischen Wirkstoff ausgewählt aus der Gruppe bestehend aus: Vitamin C, Vitamin E, Lycopen, β-Carotin, Lutein, organisches Selen, α-Liponsäure, Methylsulfonlymethan, Glutathion, Taurin, Glycin, Glutamin, Arginin, Cystein, Methionin, S-Adenosylmethionin, Nukleotiden, Nukleinsäuren, Curcumin, Grünteeextrakt, Grünschalmuschelextrakt (*Perna canaliculus*)*,* oder standardisierten Kräuterextrakten wie *Phyllanthus amarus, Fructus schisandra,* Kamille, Schwarze Johannisbeere, Teufelskralle.

17. Ein Futter umfassend eine Docosahexaensäure oder einen Ester davon, wobei der Ester der Docosahexaensäure ausgewählt ist aus der Gruppe bestehend aus: einem Triglycerid, Diglycerid, Monoglycerid, Phospholipid, Glycolipid, Sphingolipid oder Sulpholipid und mindestens einem Carotenoid, **gekennzeichnet dadurch, dass** das Carotenoid Astaxanthin ist und von einer mikrobiellen Quelle zur Verfügung gestellt wird.

18. Das Futter gemäß Anspruch 17, weiter umfassend Grünmehl.

19. Die Verwendung gemäß einem der Ansprüche 1 bis 11, wobei das Medikament ein Neutraceutical ist.

20. Die Zusammensetzung gemäß einem der Ansprüche 12 bis 15, wobei das Pharmazeutikum ein Neutraceutical ist

## Revendications

1. Utilisation d'une composition comprenant de l'acide docosahexaénoïque ou un ester de celui-ci, dans laquelle l'ester d'acide docosahexaénoïque est choisi dans le groupe constitué par : un triglycéride, diglycéride, monoglycéride, des phospholipides, un glycolipide, sphingolipide ou sulfolipide et au moins un caroténoïde, ledit caroténoïde étant l'astaxanthine et étant fourni par une source algale ou de levure, dans la fabrication d'un médicament pour le traitement d'un état inflammatoire induit par un traumatisme ou par un stress.

2. Utilisation selon la revendication 1, dans laquelle ledit acide docosahexaénoïque est fourni sous la forme d'une algue ou d'une fraction de celle-ci.

3. Utilisation selon la revendication 2, dans laquelle ladite algue est choisie dans le groupe consistant en : *Crypthecodinium ; Phaedactylum ; Isochrysis ; Schizochytrium ; Thaustochytrium ;* ou *Ulkenia.*

4. Utilisation selon la revendication 1, dans laquelle ledit acide docosahexaénoïque est fourni sous la forme d'une huile de poisson ou d'une fraction de celle-ci.

5. Utilisation selon la revendication 1, dans laquelle ladite source de levure est *Haematococcus* ou *Phaffia.*

6. Utilisation selon l'une quelconque des revendications 1 à 5, dans laquelle ledit acide docosahexaénoïque est fourni à un animal à une dose d'entre 0,05 et 500 mg/kg de poids corporel et ledit caroténoïde est fourni à une dose d'entre 0,5 et 5 000 µg/kg de poids corporel.

7. Utilisation selon l'une quelconque des revendications 1 à 5, dans laquelle ledit acide docosahexaénoïque est fourni à un animal à une dose d'entre 0,5 et 15 mg/kg de poids corporel et ledit caroténoïde est fourni à une dose d'entre 1,5 et 150 µg/kg de poids corporel.

8. Utilisation selon l'une quelconque des revendications 1 à 5, dans laquelle ledit acide docosahexaénoïque est fourni à un animal à une dose d'entre 1 et 3 mg/kg de poids corporel et ledit caroténoïde est fourni à une dose d'entre 7,5 et 22,5 µg/kg de poids corporel.

9. Utilisation selon l'une quelconque des revendications 1 à 8, dans laquelle ladite composition comprend en outre de la levure.

10. Utilisation selon l'une quelconque des revendications 1 à 9, dans laquelle ladite composition comprend en outre un agent anti-inflammatoire.

11. Utilisation selon la revendication 10, dans laquelle ledit autre agent anti-inflammatoire est choisi dans le groupe constitué par : la vitamine C, la vitamine E, le lycopène, le β-carotène, la lutéine, le sélénium organique, l'acide α-lipoïque, le méthylsulfonylméthane, le glutathion, la taurine, la glycine, la glutamine, l'arginine, la cystéine, la méthionine, la S-adénosylméthionine, les nucléotides, les acides nucléiques, la curcumine, l'extrait de thé vert, l'extrait de moule verte - (Perna canaliculus) ou des extraits d'herbes standardisés tels que *Phyllanthus amarus, Fructus Schisandra,* la camomille, la feuille de cassis, la griffe du diable.

12. Composition comprenant une source algale d'acide docosahexaénoïque et une source de levure d'astaxanthine en vue d'une utilisation comme produit pharmaceutique.

13. Composition selon la revendication 12, dans laquelle l'algue est choisie dans le groupe constitué par : *Crypthecodinium ; Phaedactylum ; Isochrysis ; Schizochytrium ; Thaustochytrium ;* ou *Ulkenia.*

14. Composition selon l'une des revendications 12 ou 13, dans laquelle la source de levure d'astaxanthine est *Haematococcus* ou *Phaffia.*

15. Composition selon l'une des revendications 12 à 14 comprenant en outre de la levure de bière inactivée.

16. Composition selon l'une quelconque des revendications 12 à 15, comprenant en outre un agent anti-inflammatoire choisi dans le groupe constitué par : la vitamine C, la vitamine E, le lycopène, le β-carotène, la lutéine, le sélénium organique, l'acide α-lipoïque, le méthylsulfonylméthane, le glutathion, la taurine, la glycine, la glutamine, l'arginine, la cystéine, la méthionine, la S-adénosylméthionine, les nucléotides, les acides nucléiques, la curcumine, l'extrait de thé vert, l'extrait de moule verte (Perna canaliculus) ou des extraits d'herbes standardisés tels que *Phyllanthus amarus, Fructus Schisandra,* la camomille, la feuille de cassis, la griffe du diable.

17. Aliment pour animaux comprenant un acide docosahexaénoïque ou un ester de celui-ci dans lequel l'ester d'acide docosahexaénoïque est choisi dans le groupe constitué par : un triglycéride, diglycéride, monoglycéride, des phospholipides, un glycolipide, sphingolipide ou sulfolipide et au moins un caroténoïde, **caractérisé par le fait que** ledit caroténoïde est l'astaxanthine et est fourni à partir d'une source microbienne.

18. Aliment pour animaux selon la revendication 17 comprenant en outre une farine de graminées.

19. Utilisation selon l'une quelconque des revendications 1 à 11, dans laquelle le médicament est un nutraceutique.

20. Composition selon l'une quelconque des revendications 12 à 15, dans laquelle le produit pharmaceutique est un nutraceutique.
